Europäisches Patentamt

**European Patent Office**

Office européen des brevets

(11) Publication number: **0 209 924**
**A1**

(12) **EUROPEAN PATENT APPLICATION**

(21) Application number: **86201027.9**

(22) Date of filing: **13.06.86**

(51) Int. Cl.⁴: **C 08 B 37/10**
**A 61 K 31/73**

(30) Priority: **12.07.85 NL 8502009**

(43) Date of publication of application:
**28.01.87 Bulletin 87/5**

(84) Designated Contracting States:
**BE CH DE FR GB IT LI NL SE**

(71) Applicant: **AKZO N.V.**
**Velperweg 76**
**NL-6824 BM Arnhem(NL)**

(72) Inventor: **van Dedem, Gijsbert Willem Karel**
**Bachlaan 23**
**NL-5384 BL Heesch(NL)**

(72) Inventor: **van Houdenhoven, François Egbert**
**Rijksweg 125 B**
**NL-5384 AH Heesch(NL)**

(72) Inventor: **Sanders, Adrianus Lambertus**
**Steeuwichtweg 8**
**NL-5406 PP Uden(NL)**

(72) Inventor: **Meuleman, Dirk Gerrit**
**Beethovengaarde 69**
**NL-5344 CC Oss(NL)**

(72) Inventor: **Moelker, Huibert Cornelis T.**
**Torenstraat 7**
**NL-5366 BJ Megen(NL)**

(74) Representative: **Douma, Anno Do et al,**
**Organon International B.V. Postbus 20**
**NL-5340 BH Oss(NL)**

(54) New anti-trombosis agent based on glycosaminoglycan, process for its preparation, and pharmaceutical compositions.

(57) The present invention is concerned with a new anti-thrombosis agent based on a mixture of glycosaminoglycans having

a) a mean molecular weight of between 5,000 and 9,000 daltons;

b) a nitrogen content of 2 to 3% by weight;

c) a sulphur content of 8 to 12% by weight;

d) a content of N-sulphate groups, in milliequivalents/g, of 1.2 to 1.6;

e) a content of O-sulphate groups, in milliequivalents/g, of 1.7 to 2.1;

f) a content of carboxyl groups, in milliequivalents/g, of 1.6 to 2.0;

g) a content of N-acetyl groups, in milliequivalents/g, of 0.3 to 0.6;

h) a galactosamine content, in milliequivalents/g, of 0.0 to 0.1;

i) an iduronic acid content, in mmol/g, of 1.2 to 1.4 and

j) a glucuronic acid content, in mmol/g, of 0.3 to 0.5, with methods for its preparation and with pharmaceutical compositions comprising the above anti-thrombosis agent. The anti-thrombosis agent according to the present invention exhibits an improved benefit/risk ratio with respect to the anti-thrombosis activity relative to the haemorrhagic activity.

EP 0 209 924 A1

- 1 -

# NEW ANTI-THROMBOSIS AGENT BASED ON GLYCOSAMINOGLYCAN, PROCESS FOR ITS PREPARATION, AND PHARMACEUTICAL COMPOSITIONS.

The invention relates to a new anti-thrombosis agent based on a heterogeneous mixture of glycosaminoglycans and to processes for its preparation.

The invention also relates to pharmaceutical compositions which contain the said mixture. The invention in particular relates to a glycosaminoglycan anti-thrombosis agent having a high affinity for anti-thrombin III (AT III).

It is known that certain mucopolysaccharides influence the clotting properties of blood. The best-known mucopolysaccharide is heparin (a heterogeneous mixture of glucosaminoglycans), which is used in the prophylaxis and treatment of venous thrombosis and thrombo-embolisms. The anti-thrombosis action of heparin is based on the acceleration of the inhibition of blood-coagulating factors by anti-thrombin III. A great problem in the use of heparin for the prevention and in the therapy of thrombosis and thrombo-embolisms is the property of heparin to cause haemorrhaging. The risk of haemorrhages can be reduced somewhat by optimising the dose, manner and frequency of administration but the ratio of anti-thrombosis properties to haemorrhage-inducing properties of heparin can barely be influenced. A further shortcoming of heparin is its rather short duration of action, so that for good prophylaxis a dose usually has to be administered at least twice per twenty four hours.

Various attempts have already been made to prepare "improved heparins" or to develop heparinoids having improved properties. Thus, for example, British patent publication 2,002,406 relates to an "improved heparin" consisting of an oligo-heteropolysaccharide mixture having a molecular weight distribution of between 2000 and 5000 daltons, which mixture is obtained by depolymerisation of heparin or is isolated from the mother liquors of the normal preparation of heparin, after which the material thus obtained is additionally sulphated. The ratio of anti-thrombosis action to anti-coagulant action of the material thus obtained is supposed to be more advantageous than in the case of heparin.

Various other "improved heparins" with a low molecular weight (LMW-heparins) have already been prepared. See, for example, U.S. Patent Specifications 3,766,167, 4,281,108, 4,303,651, 4,351,938, 4,396,762, 4,401,662, 4,401,758, 4,438,261 and 4,474,770.

A heparinoid with improved properties is described in EP-A-0,066,908. This product shows a significant dissociation between anti-thrombosis action and haemorrhagic action.

A new anti-thrombosis agent has now been found, which is a fraction of the product described in EP-A-0,066,908 and exhibits high affinity for AT III.

The product described in EP-A-0,066,908 is hereinafter referred to as HEF and the new product as HA-HEF.

HA-HEF also consists of a mixture of oligosaccharides and (hetero)-polysaccharides (glycosaminoglycans), which are built up of hexose derivatives, in the main glucuronic acid, iduronic acid, glucosamine and sulphated and/or acetylated derivatives thereof, with uronic acid derivatives and glucosamine derivatives alternating with one another.

It is a white, amorphous powder having a mean molecular weight between 5000 D and 9000 D, more specifically between 6000 D and 8000 D, determined relative to dextran by means of gel permeation chromatography. The product can contain a small quantity of chondroitin-sulphuric acid (max. 2% by weight) and dermatansulphate (max. 2% by weight). The galactosamine content is between 0 and 0.1 mmol/g and is usually less than 0.075 mmol/g. The nitrogen content is between 2 and 3% by weight and is usually about 2.5% by weight; the sulphur content is between 8 and 12% by weight and is usually 10-10.5% by weight; the content of N-sulphate groups in milliequivalents/g is 1.2-1.6; the content of O-sulphate groups in milliequivalents/g is 1.7-2.1; the content of carboxyl groups in milliequivalents/g is between 1.6 and 2.0; the content of N-acetyl groups in milliequivalents/g is between 0.3 and 0.6; the iduronic acid content in mmol/g is between 12 and 1.4 and the glucuronic acid content in mmol/g is between 0.3 and 0.5.

The pharmacological profile of HA-HEF is characterised by

1) an anti-clotting activity (USP) of less than 10 international units per mg (IU/mg) and hence only a fraction (usually less than 5%) of that of heparin USP;

2) an anti-thrombin activity which is less than 5% of that of heparin USP;

3) an anti-$X_a$ activity of 350-400 U/mg (2 to 3 times that of heparin USP);

4) an anti-thrombosis activity (Umetsu model) with an $ID_{50}$ of about 0.2 to 0.4 mg/kg i.v.;

5) a "benefit/risk" ratio which, surprisingly, is even more advantageous than that of HEF with respect to the anti-thrombosis activity relative to the haemorrhagic activity and is 15-50 times more advantageous than that of heparin USP; and

6) a half-life which is roughly equal to that of HEF, which means that it is at least twice as long as that of heparin USP.

Further the haemorrhagic activity of HA-HEF increases only slightly over a large dosage range and is even lower than that of HEF, while the haemorrhagic activity of heparin USP at a dosage of 1 mg/kg i.v. is already clearly noticeable, and rapidly increases at higher dosages.

This interesting profile makes HA-HEF very suitable for the prophylaxis and treatment of venous thrombosis and thrombo-embolisms. The product is, above all, particularly suitable for the prophylaxis of DVT (deep vein thrombosis) in patients who undergo, or have undergone, a hip operation. Low doses (s.c.) of heparin are ineffective for that purpose and higher doses are contra-indicated because of the high risk of haemorrhaging.

HA-HEF appears not to have mutagenic or toxic properties, even at high doses (up to 3200 anti-$X_a$-U/kg/day).

HA-HEF can be obtained from HEF in various ways. A feature common to these methods of preparation is that HEF is brought into contact with AT III. The part of HEF complexed with AT III is thereafter separated from the non-complexed part, after which HA-HEF is liberated from the complex obtained and is subsequently isolated.

The AT III used can be of human origin but also of animal origin, for example AT III isolated from cattle plasma.

HA-HEF can be prepared batchwise or with the aid of columns.

For example, AT III can be added to a buffered (pH 7.0-8.0) aqueous solution of HEF. The mixture is stirred for some time, preferably at an elevated temperature (37 °C). The AT III complex formed is thereafter separated from HEF by ultrafiltration, gel permeation chromatography or ion exchange chromatography and is decomposed by means of a buffered salt solution, after which the HA-HEF is isolated.

A more advantageous embodiment of this process is that in which the AT III is used covalently bonded to a carrier material, for example agarose or a suitable silica-based carrier material. The complexed part of the HEF is then simpler to separate from the non-complexed part, for example by filtering off with suction on a glass filter.

AT III covalently bonded to carrier material can also be used in column form, over which the HEF-containing solution is then passed. In this method, the separation of complexed and non-complexed HEF is even simpler. A solution of HEF, buffered to a pH of between 7 and 8 with an acetate, phosphate or TRIS-HCl buffer is passed over an AT III column which has beforehand been equilibrated with the same buffer. HA-HEF is bonded to the column. After the column has been washed, HA-HEF is eluted from the column by means of a salt solution, for example a 1-3-molar solution of LiCl, NaCl or CaCl$_2$. The eluate is desalinated by dialysis or ultrafiltration and the HA-HEF is isolated by methanol precipitation or freeze-drying.

The methods of preparation mentioned are known per se for heparin. See, in this context, for example U.S. Patent Specifications 4,119,774 and 4,301,153. See also, for example, FEBS letters 66 (1976), 90-93 and Biochem. and Biophys. Res. Comm. 69 (1976), 570-577.

Whenever heparin is treated in this manner, a heparin fraction with high affinity for AT III (HA-heparin) is obtained. The yield of HA-heparin is about 25-35% by weight. In the case of HA-HEF from HEF, the yield is about 2-7% by weight.

HA-heparin has an anti-clotting activity (USP) which is 2-3 times as great as that of heparin. In the case HA-HEF, this activity is hardly altered compared to that of HEF.

The anti-X$_a$ activity of HA-heparin is about twice that of heparin. HA-HEF, however, has an anti-X$_a$ activity which is 30-100 times as great as that of HEF.

The "benefit/risk" ratio with respect to the anti-thrombosis activity relative to the haemorrhagic activity is found to be little different for HA-heparin from that of heparin, in other words HA-heparin retains the unfavourable haemorrhage-inducing properties of heparin. The "benefit/risk" ratio of HA-HEF is, however, found to be even more advantageous than that of HEF.

6

The new HA-HEF can be converted, in the manner customary for heparin, to a pharmaceutical dosage form, for example by dissolution in water suitable for injection purposes, to which, if desired, pharmaceutically acceptable auxiliaries (preservatives and certain salts) can be added. Clinical use is by subcutaneous or intravenous (if appropriate, intermittent) injection or by infusion. Other dosage methods, such as intrapulmonary administration via spray inhalation, or administration via the skin by means of an ointment or cream, or via mucous membranes, for example by means of a suppository, are also possible.

The invention is illustrated by the examples and tests which follow, without these implying any limitation.

## Example 1
### Preparation of immobilised AT III on a silica-based carrier material

10 g/l of purified heparin were added to a buffered solution of cattle AT III (10 mg/ml: 0.1 M phosphate buffer, pH 7.8) and thereafter 500 g/l of a silica-based carrier material, as described in EP-A-0,043,159 and containing N-hydroxysuccinimide groups, were added. The mixture was stirred for 16 hours at room temperature, and the carrier material was filtered off and washed with 0.1 M phosphate buffer (pH 7.8) and subsequently with 0.1 M ethanolamine. Thereafter the material was incubated for 5 hours with ethanolamine (0.1 M) at 0-5 "C, again washed with 0.1 M phosphate buffer (pH 7.8) and subsequently treated with 3M NaCl in 0.1 M phosphate buffer to remove the heparin.

7

## Example 2

### Preparation of HA-HEF

A column of the immobilised AT III obtained according to Example 1 was equilibrated with an 0.02 M phosphate buffer (pH 7.4). A buffer solution of HEF (20 g/l) was passed through this column (50 ml per litre of column material). The column was then washed with 0.02 M $NH_4$-acetate buffer (pH 8.0) for 1 hour and then with 0.4 M NaCl in the same buffer, again for one hour. Thereafter the column was eluted with 0.02 M $NH_4$-acetate buffer (pH 8.0) containing 3 M of NaCl. The eluate was desalinated and at the same time concentrated to a concentration of at least 10 mg/ml by means of a hollow fibre ultrafiltration device. The HA-HEF was precipitated from the concentrated solution by means of 75% methanol. Yield 3.6% by weight.

The HA-HEF obtained had a mean molecular weight of 7100 D, a sulphur content of 10.3% by weight and a nitrogen content of 2.45% by weight, and contained 1.36 milliequivalents/g of N-sulphate, 1.87 milliequivalents/g of O-sulphate, 1.75 milliequivalents/g of carboxyl, 0.422 milliequivalents/g of N-acetyl, 1.33 mmol/g of iduronic acid, 0.42 mmol/g of glucuronic acid and 0.07 mmol/g of galactosamine.

## Example 3

### Preparation of HA-HEF

A column of AT III immobilised on agarose gel matrix was equilibrated with 0.05 M TRIS-HCl (0.1 M NaCl) buffer (pH 7.5). A solution of HEF (20 g/l) in the same buffer was passed through this column (50 ml per litre of column material). The column was then washed with the above mentioned buffer. Thereafter, it was eluted with 0.05 M TRIS-HCl buffer containing 1 M $CaCl_2$ (pH 7.2). The eluate was desalinated by gel filtration over a dextran gel matrix column and the salt-free solution obtained was freeze-dried.

0209924

8

The HA-HEF obtained had a mean molecular weight of 6600 D, a sulphur content of 10.1% by weight and a nitrogen content of 2.35% by weight, and contained 1.31 milliequivalents/g of N-sulphate, 1.82 milliequivalents/g O-sulphate, 1.78 milliequivalents/g of carboxyl, 0.403 milliequivalents/g of N-acetyl, 1.29 mmol/g of iduronic acid, 0.47 mmol/g of glucuronic acid and 0.04 mmol/g of galactosamine.

## Pharmacological tests

The HA-HEF of Example 2 was tested pharmacologically in comparison with HEF (prepared according to Example 3 of EP-A-0,066,908), heparin and HA-heparin.

## Effect on blood clotting

| Product | Anti-clotting activity (IU/mg) | Anti-thrombin activity (IU/mg) | Anti-$X_a$ activity (IU/mg) |
|---|---|---|---|
| HA-HEF | 5 | 1 | 370 |
| HEF | 3 | 0.2 | 8 |
| heparin | 175 | 161 | 166 |
| HA-heparin | 370 | 437 | 284 |

The anti-clotting activity was determined in accordance with the USP method. The anti-thrombin activity and the anti-$X_a$ activity were determined in accordance with the chromogenic substrate method, using purified cattle anti-thrombin III and the substrates S-2238 and S-2222 respectively.

## Anti-thrombosis activity

The anti-thrombosis activity was determined in the Umetsu model (Thrombosis Research $\underline{27}$, (1982) 353-363) in the rat.

### Results

| Product | $ID_{50}$ in U/kg |
|---|---|
| HA-HEF | 102 |
| HEF | 45 |
| heparin | 50 |
| HA-heparin | 55 |

## Haemorrhagic activity

a) Subdermal haemorrhaging test in the rat.

The product to be tested was administered intravenously. After 1 minute, haemorrhaging was induced by making an incision in the neck of an anaesthetized rat.

The blood lost in 30 minutes was collected in 20 ml of water. The haemoglobin concentration in the water was measured spectrophotometrically and used as a parameter of the blood loss (see Journal of Biochemistry $\underline{2}$, (1983), 173).

### Results

| Product | a) TD in U/kg (1) |
|---|---|
| HA-HEF | 1535 |
| HEF | 510 |
| heparin | 170 |
| HA-heparin | 200 |

(1) TD= dose at which triplication of the haemorrhaging effect occurs.

10

The data indicate a much lower haemorrhaging tendency for HA-HEF, which is even more than twice as advantageous as that of HEF. HA-heparin has, admittedly, a lower haemorrhaging tendency than heparin but the improvement is by far not as striking as in the case of HA-HEF compared to HEF.

## Half-life of the anti-thrombosis action

The duration of the anti-thrombosis action was determined, in the Umetsu model, for HA-HEF (product of Example II), HEF and heparin. The result was as follows:

| Product | $T_{\frac{1}{2}}$ (anti-$X_a$) in the rat, in hours |
|---------|--------------------------------------------------|
| HA-HEF  | 2-5 |
| HEF     | 2-5 |
| heparin | 0.5-1 |

*1*

CLAIMS

1.    New anti-thrombosis agent based on a mixture of glycosamino-
glycans having
   a) a mean molecular weight of between 5,000 and 9,000 daltons,
   b) a nitrogen content of 2 to 3% by weight,
   c) a sulphur content of 8 to 12% by weight,
   d) a content of N-sulphate groups, in milliequivalents/g, of 1.2 to
      1.6,
   e) a content of O-sulphate groups, in milliequivalents/g, of 1.7 to
      2.1,
   f) a content of carboxyl groups, in milliequivalents/g, of 1.6 to
      2.0,
   g) a content of N-acetyl groups, in milliequivalents/g, of 0.3 to
      0.6,
   h) a galactosamine content, in milliequivalents/g, of 0.0 to 0.1,
   i) an iduronic acid content, in mmol/g, of 1.2 to 1.4 and
   j) a glucuronic acid content, in mmol/g, of 0.3 to 0.5.

2.  Anti-thrombosis agent according to claim 1 having an anti-clotting activity (USP) of less than 10 international units per mg.

3.  Anti-thrombosis agent according to claims 1-2 having an anti - thrombin activity which is less than 5% of that of heparin USP.

4.  Anti-thrombosis agent according to claims 1-3 having an anti-$X_a$ activity of 350-400 U/mg.

5.  Anti-thrombosis agent according to claims 1-4 having an anti-thrombosis activity (Umetsu model) with an $ID_{50}$ of about 0.2 to 0.4 mg/kg i.v..

6.  Anti-thrombosis agent according to claims 1-5 having a "benefit/ risk" ratio which is 15-50 times more advantageous than that of heparin USP in respect of the anti-thrombosis activity in relation to the haemorrhagic activity.

7.  Anti-thrombosis agent according to claims 1-6 having a half-life which is at least twice as long as that of heparin USP.

8.  Anti-thrombosis agent according to claims 1-7, obtainable from the product described in EP-A-0,066,908 by affinity chromatography using AT III.

9.  Process for the preparation of an anti-thrombosis agent according to one or more of the preceding claims, characterised in that the product described in EP-A-0,066,908 is used as the starting material, this product is brought into contact with AT III, the complex formed with AT III is seperated from the non-complexed product, the complex is decomposed and the mixture of glycosaminoglycans thus obtained is isolated.

10. Pharmaceutical composition characterised in that it contains the anti-thrombosis agent of one or more of claims 1-9.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | EP-A-0 064 452 (CHOAY) <br> * Page 10, lines 20-30; claims * | 1-10 | C 08 B 37/10 <br> A 61 K 31/73 |
| Y | EP-A-0 048 898 (CUTTER) <br> * Page 11, lines 11-13; claims * | 1-10 | |
| Y | THROMBOSIS RESEARCH, vol. 27, 1982, pages 679-690, Pergamon Press Ltd., US; P.A. OCKELFORD et al.: "Comparison of the in vivo hemorrhagic and antithrombotic effects of a low antithrombin-III affinity heparin fraction" <br> * Pages 683,684,688 * | 1-10 | |
| Y | EP-A-0 040 144 (PHARMINDUSTRIE) <br> * Page 5, table A * | 1-10 | TECHNICAL FIELDS SEARCHED (Int Cl 4) <br><br> C 08 B <br> A 61 K |

The present search report has been drawn up for all claims

| Place of search | Date of completion of the search | Examiner |
|---|---|---|
| THE HAGUE | 09-10-1986 | LENSEN H.W.M. |